(19) Europäisches Patentamt
European Patent Office
Office européen des brevets

(11) Publication number: 0 153 858
A2

(12) # EUROPEAN PATENT APPLICATION

(21) Application number: 85301299.5

(22) Date of filing: 26.02.85

(51) Int. Cl.[4]: A 61 K 31/557
//C07C177/00

(30) Priority: 29.02.84 US 584672
26.10.84 US 665252
31.01.85 US 696940

(43) Date of publication of application:
04.09.85 Bulletin 85/36

(84) Designated Contracting States:
BE CH DE FR GB IT LI NL SE

(71) Applicant: THE UPJOHN COMPANY
301 Henrietta Street
Kalamazoo, Michigan 49001(US)

(71) Applicant: THE REGENTS OF THE UNIVERSITY OF CALIFORNIA
2199 Addison Street
Berkeley California 94720(US)

(72) Inventor: McCarter, Douglas Eugene
3126 Catawba Court
Pleasanton California 94566(US)

(72) Inventor: Holcroft, James Walter
3311 S. El Macero Drive
El Macero California 94618(US)

(74) Representative: Perry, Robert Edward et al,
GILL JENNINGS & EVERY 53-64 Chancery Lane
London WC2A 1HN(GB)

(54) **The therapeutic use of prostaglandins.**

(57) The use of a PGE-like prostaglandin for the manufacture of a medicament for the treatment or prevention of a human suffering from or prone to acute respiratory distress syndrome or multiple vital organ damage, and for the treatment of a human suffering from shock, trauma, sepsis or any combination thereof.

EP 0 153 858 A2

Croydon Printing Company Ltd.

# THE THERAPEUTIC USE OF PROSTAGLANDINS

The present invention provides a new use of known pharmacological agents. In particular, this invention relates to method for treating patients with acute respiratory distress syndrome (ARDS - sometimes known as adult respiratory distress syndrome) resulting from shock, trauma or sepsis and preventing death resulting from multiple organ damage in this disease. ARDS seems to be secondary to embolization to the lungs of microaggregates of platelets-and/or white cells. The syndrome, and the associated multiple organ failure, accounts for most of the noncerebral deaths of patients who have been resuscitated from shock, trauma, and sepsis or the other inciting events of Table I. The pulmonary failure is characterized by ventilation-perfusion mismatching with low arterial oxygen concentrations, poor lung compliance, and in some cases, increased pulmonary vascular resistance. In addition to damage to the lungs, damage to other organs is associated with shock, trauma, and sepsis. Overall mortality in patients with ARDS or other severe organ failure, after shock, trauma, or sepsis, is over 50%.

Current treatment of ARDS concentrates on treatment of the underlying shock, trauma, or sepsis. Rapid resuscitation from shock, rapid control of bleeding, early institution of mechanical ventilation with a volume ventilator, and titration of intravascular volume, sometimes using a Swan-Ganz pulmonary arterial catheter, help in treatment of shock. Immobilization of fractures, debridement of dead or devitalized tissue, drainage of pus, closure of holes in the gastrointestinal tract, diversion of the gastrointestinal tract proximal to damage, and administration of antibiotics can all help prevent the adverse effects of tissue

trauma or infection. Only a few specific treatments, however, are available to treat ARDS or other organ failure once it develops. In those cases where patients are suffering from severe cases of ARDS, large doses of heparin therapy may be given in an attempt to reduce intravascular clotting and microembolization to the lungs. Heparin, however, increases the risk of additional bleeding in patients suffering from severe injuries. The method proposed here would treat ARDS and other organ failure after shock, trauma,or sepsis without risks of this sort.

This invention also relates to a method for treating patients who are at risk for developing failure of vital organs (lungs, liver, kidney, gastrointestinal tract, brain, or heart) as a result of a catastrophic, traumatic, or septic event. These inciting catastrophic events have been extensively described for ARDS and some are listed in Table I. These are the events which place the patients treated by this invention at risk for vital organ failure.

Like ARDS, failure of other vital organs may be secondary to the embolization and/or deposition of platelets and/or white blood cells to or in the liver, kidney, gastrointestinal tract, brain, or heart. Liver and kidney failure are usually noted initially by the appearance of pathologic levels of certain cell products in the blood (bilirubin and creatinine, for example). Failure of the gastrointestinal tract may present with gastrointestinal bleeding. Brain dysfunction may present with disorientation. Heart dysfunction may present with congestive heart failure associated with low cardiac outputs and high end diastolic ventricular filling pressures. Bilirubin levels above 5 mg % and creatinine levels above 2 mg % indicate failure of the liver or kidney. Gastrointestinal hemorrhage requiring transfusion represents failure of the gastrointestinal tract. Severe disorientation to person, time, or place represent brain dysfunction. A cardiac index less than 3.3 $liters{\cdot}min^{-1}{\cdot}m^{-2}$ associated with right atrial pressures greater than 8 mm Hg or left atrial pressures greater than 15 mm Hg represent failure of the heart (these pressures are measured with respect to atmosphere with the pressure transducer located at the level of the mid thorax with the pressures being measured at end expiration).

The patient population to be treated by the prostaglandins of this invention include those who have evidence of organ failure, diagnosed as previously described (respiratory distress, embolization and/or deposition of platelets or white cells to the vital organs, high levels of blood bilirubin or creatinine, gastrointestinal hemorrhage, disorientation, decreased cardiac index, etc. as a result of the inciting events such as those listed on Table 1) or who are at significant risk for organ failure as a result of the acute inciting events such as those of Table 1. These risks are generally known to those skilled in the art and include the following:

(1) Any septic or traumatic insult that results in the patient requiring ventilatory support (This is evidence of beginning lung failure.);
(2) Documented peritonitis;
(3) Lung contusion or major thoracic trauma;
(4) Major non-thoracic trauma;
(5) An operative procedure requiring 6 or more units of blood in the perioperative period;
(6) Second or third degree burns involving about 30% or more of the patient's body;
(7) Significant fat embolization resulting from trauma or surgery;
(8) Hemorrhagic or septic shock;
(9) Cardiogenic or anaphylactic shock;
(10) Severe viral, bacterial or fungal pneumonia;
(11) Inhalation of oxygen (greater than 50% $O_2$), smoke or nitrous oxide (for an extended period of time);
(12) Aspiration of gastric contents into the lungs;
(13) Drug overdose;
(14) Disseminated intravascular coagulation;
(15) Near drowning (fresh or salt water);
(16) Pancreatitis or uremia;
(17) Patients requiring long periods on cardiopulmonary bypass;
(18) Patients whose heart has stopped for a prolonged period (greater than 3 minutes);
(19) Air or amniotic fluid embolization; and

(20) Any patient who requires multiple transfusions of 6 or more units of blood.

See, also, e.g., Pepe, et al., "Clinical Predictors of the Adult Respiratory Distress Syndrome," American Journal of Surgery, 144:124 (1982).

The prostaglandins used in the present invention are derivatives of prostanoic acid. A trivial system of nomenclature has been devised, which classifies the prostaglandins according to the substituents on the cyclopentane ring. See, N.A. Nelson, Journal of Medicinal Chemistry, 17:911 (1974). For a further discussion of the prostaglandins, see Bergstrom, et al., Pharmacol. Rev. 20:1 (1968), and references cited therein.

The term prostaglandin analog herein refers to those compounds structurally related to the prostaglandins (in that they exhibit a cyclopentane, or adjacently homologous cycloalkane, ring and a pair of side chains attached to adjacent carbon atoms of the ring) which retain characteristic biological properties of the prostaglandins. See Bergstrom, cited above. Various structural modifications of the prostaglandins are known to produce useful prostaglandin analogs. Many of these structural modifications are discussed in the U.S. patents set forth below.

Prostaglandin $E_1$ ($PGE_1$), $PGE_2$ and $PGI_2$ are known derivatives of prostanoic acid.

$PGE_1$, its lower alkyl esters, salts and amides are known to be potent pharmaceutical agents. The use of $PGE_1$ and its alkyl esters, salts and amides for the treatment of peripheral vascular disease by non-intrarterial administration is described in U.S. Patent 4,103,026. In U.S. Patent 4,116,988 various uses of $PGE_1$ are described. They include stimulation of smooth muscles; antilipolytic activity; stimulation of epidermal proliferation and keratinization; treatment of nasal congestion; treatment of bronchial asthma; bronchitis; bronchiectasis; pneumonia and emphysema; use as additives to blood, blood products, blood substitutes and other fluids which are used in artificial extracorporeal circulation and perfusion of isolated body parts; and induction of labor in female animals.

$PGI_2$, also known as prostacyclin, and its pharmaceutically acceptable salts, esters and amides have exhibited potent pharmacological activity. See U.S. Patents 4,158,667 and 4,338,325 which discloses their utility as platelet aggregate inhibitors. 6a-Carba-prostacyclin (carbacyclin) and the 9-β-methyl derivative thereof, are disclosed in U.S. patents 4;238,414 and 4,420,632, respectively.

Numerous other prostaglandins and their analogs are known. Thus, PGE1 is disclosed in U.S. Patent 3,069,322 and PGE2 is disclosed in U.S. patent 3,598,858. PGE2 esters are disclosed in U.S. patents 3,795,697 and 3,691,216. 20-Isopropylidene compounds are disclosed in Japanese Kokai 77-97946. Other prostaglandin analogs are disclosed in U.S. patents 3,069,322; 3,598,858; 3,636,120; 3,639,463; 3,691,216; 3,706,789; 3,725,454; 3,726,909; 3,728,382; 3,759,978; 3,776,938; 3,776,939; 3,795,697; 3,804,879; 3,804,889; 3,804,890; 3,812,172; 3,812,179; 3,813,433; 3,836,578; 3,839,409; 3,849,487; 3,852,337; 3,855,270; 3,880,912; 3,888,916; 3,890,372; 3,894,062; 3,929,861; 3,929,862; 3,959,319; 3,962,293; 3,968,140; 3,969,376; 3,969,377; 3,969,380; 3,974,189; 3,974,195; 3,987,084; 3,998,867; 3,998,869; 4,005,133; 4,017,535; 4,032,576; 4,057,564; 4,060,534; 4,067,891; 4,069,386; 4,081,471; 4,082,783; 4,084,063; 4,098,805; 4,103,098; 4,119,666; 4,128,577; 4,130,584; 4,130,720; 4,130,721; 4,139,564; 4,158,667; 4,171,460; 4,176,236; 4,197,257; 4,205,178; 4,220,796; 3,903,131; 3,954,741; 4,187,381; 3,972,917; 4,052,512; 3,823,180; 4,107,191; 4,147,879; and 4,165,436.

Reversal by $PGE_1$ of the increase of microvascular permeability in sheep has been observed. Staub, N.C., "Pulmonary Edema: Physiologic Approaches to Management", Chest, Vol. 74, pp. 559-64 (Nov. 1978). Increased lung microvascular permeability has been associated with respiratory failure in endotoxin-induced lung injury in sheep. Smith, Michael E. et al, "Prostaglandin $E_1$ and Prostacyclin Infusion Decrease Lung Injury", Journal of Surgical Research Vol. 32 (3), 283-288, (March 1982). It has also been reported that many consider endotoxin-induced lung injury to be the same as that seen clinically in sepsis. It has been stated that lung and systemic injury associated with endotoxin infusion has been decreased by the infusion of $PGE_1$ and $PGI_2$. "Role of Prostacyclins in Acute Pulmonary Microvascular Injury", Demling,

R.H., Annals New York Academy of Science, Vol. 384, pp. 517-34 (1982). All of the reported studies, prior to this invention, relating to the use of $PGE_1$ or $PGI_2$ to reduce microvascular permeability involve the treatment of animals in endotoxin-induced lung injury. In most of the studies endotoxin-induced lung injury was induced in sheep by injecting them with E. coli endotoxin and $PGE_1$ and $PGI_2$ were infused at rates of over 100 ng/kg/min and over 10/ng/kg/min., respectively. See Demling et al, "The Effect of Prostacyclin Infusion on Endotoxin-Induced Lung Injury", Surgery, Vol. 89, No. 2, pp. 257-263 (Feb. 1981). Ogletree, M. L. et al, "$PGE_1$ Reduces Lung Transvascular Filtration During Endotoxin Induced High Permeability in Sheep", Clinical Research, Vol. 27, No. 2, p. 402A, April 1979. Demling, et al, "The Effect of Prostacyclin Infusion on Endotoxin-Induced Lung Injury", Surgery, St. Louis, Vol. 89, No. 2, pp. 257-263 (Feb. 1981). Raflo, G.T. et al, "Mechanism of the Protective Effects of Prostaglandin E1 and F2a in "Canine Endotoxin Shock", European Journal of Pharmacology, 24, pp. 86-95 (1973). The survival rate of beagles suffering from hemorrhagic shock was improved by infusion with $PGE_1$. Machiedo et al, "Comparison of Corticosteroids and Prostaglandins in Treatment of Hemorrhagic Shock", Annals of Surgery, Vol. 190, No. 6, pp. 735-39, (Dec. 1979). $PGI_2$ has been reported experimentally to be beneficial in increasing the survival rate after trauma. See, e.g., Lefer et al, "Beneficial Actions of Prostaglandins in Traumatic Shock", Prostaglandins 17:761-7, 1979. Breen et al, "The Effects of $PGE_1$ and Ibuprofen on Endotoxin-Induced Pulmonary Dysfunction: A Biochemical and Electron Microscope Study", Circ. Shock, 9, (2), 197 (1982); H.B. Hechtman, et al, "The Management of Cardiorespiratory Failure in Surgical Patients", Advances in Surgery, Ed LD McLean, Vol. 15 (1981), pp. 123-156; and Lefer, "Role of Prostaglandins and Thromboxanes in Shock States," Handbook of Shock and Trauma, Vol. I: Basic Science (New York 1983).

P.L. Appel and W.C. Shoemaker, in an abstract in the Symposium Syllabus for the Critical Care Medicine 21st Annual Symposium (March 3-6, 1983) and in Hemodynamic and Oxygen Transport Effects of Prostaglandin $E_1$ in Patients with Adult Respiratory Distress Syndrome, Critical Care Medicine, Vol. 12, No. 6, pp. 528-529, 1984, described the administration of $PGE_1$ to 5 patients with severe postoperative ARDS. While

it is reported that the lung function of each patient improved, it is also noted that three patients died of associated problems, including vital organ failure.

According to the present invention, a PGE-like prostaglandin is used to prepare a medicament for use (especially human surgical or therapeutic treatment) in treating or preventing ARDS, in treating or preventing multiple organ failure in a human having or prone to ARDS or organ failure, or in treating or preventing any one or combination of shock, trauma and sepsis.

The medicament may be known per se. It may comprise the active ingredient and a pharmaceutically acceptable carrier or excipient. The medicament (or pharmaceutical composition) may be provided in a container. It may be in unit dosage form, e.g. a discrete solid or encapsulated liquid form. The amount of the active ingredient, the PGE-like prostaglandin, in use or in a dosage unit should be sufficient for the given purpose, e.g. to be effective in the given use for at least 7 days.

The PGE-like prostaglandin may have the formula

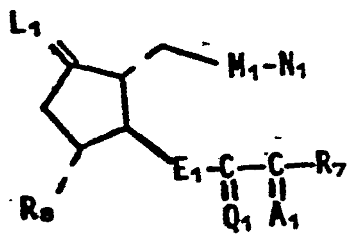

I

wherein $N_1$ is

(1) $-COOR_1$, wherein $R_1$ is

(a) hydrogen,

(b) $(C_1-C_{12})$alkyl,

(c) $(C_1-C_{10})$cycloalkyl,

(d) $(C_6-C_{16})$aralkyl,

(e) phenyl, optionally substituted by one, 2 or 3 substituents independently selected from chloro and $(C_1-C_3)$alkyl,

(f) phenyl substituted in the para position by

(i) $-NH-CO-R_{25}$,

(ii) $-CO-R_{26}$,

(iii) $-O-CO-R_{54}$ or

(iv) $-CH=N-NH-CO-NH_2$ wherein $R_{25}$ is methyl, phenyl, acetamidophenyl, benzamidophenyl, or $-NH_2$; $R_{26}$ is methyl, phenyl, $-NH_2$, or methoxy; and $R_{54}$ is phenyl or acetamidophenyl; inclusive, or

(g) a pharmacologically acceptable cation;

(2) $-CH_2OH$,

(3) $-COL_4$, wherein $L_4$ is

(a) amino of the formula $-NR_{51}R_{52}$, wherein $R_{51}$ and $R_{52}$ are

(i) hydrogen

(ii) $(C_1-C_{12})$alkyl,

(iii) $(C_3-C_{10})$cycloalkyl,

(iv) $(C_7-C_{12})$aralkyl,

(v) phenyl, optionally substituted with one, 2 or 3 chloro, $(C_1-C_3)$alkyl, hydroxy, carboxy, $(C_2-C_5)$-alkoxycarbonyl, or nitro,

(vi) $(C_2-C_5)$carboxyalkyl,

(vii) $(C_2-C_5)$carbamoylalkyl,

(viii) $(C_2-C_5)$cyanoalkyl,

(ix) $(C_3-C_6)$acetylalkyl,

(x) $(C_7-C_{11})$benzoalkyl, optionally substituted by one, 2 or 3 chloro, $(C_1-C_3)$alkyl, hydroxy, $(C_1-C_3)$-alkoxy, carboxy, $(C_2-C_5)$alkoxycarbonyl, or nitro,

(xi) pyridyl, optionally substituted by one, 2 or 3 chloro, $(C_1-C_3)$alkyl, or $(C_1-C_3)$alkoxy,

(xii) $(C_6-C_9)$pyridylalkyl optionally substituted by one, 2 or 3 chloro, $(C_1-C_3)$alkyl,

(xiii) $(C_1-C_4)$hydroxyalkyl,

(xiv) $(C1-C4)$dihydroxyalkyl,

(xv) $(C_1-C_4)$trihydroxyalkyl,

with the further proviso that not more than one of $R_{51}$ and $R_{52}$ is other than hydrogen or alkyl,

(b) cycloamino selected from the group consisting of pyrrolidino, piperidino, morpholino, piperazino, hexamethyleneimino, pyrrolino, or 3,4-didehydropiperidinyl

optionally substituted by one or 2 $(C_1-C_{12})$alkyl of one to 12 carbon atoms, inclusive,

(c) carbonylamino of the formula $-NR_{53}COR_{51}$, wherein $R_{53}$ is hydrogen or $(C_1-C_4)$alkyl and $R_{51}$ is other than hydrogen, but otherwise as defined above,

(d) sulfonylamino of the formula $-NR_{53}SO_2R_{51}$, wherein R51 and $R_{53}$ are as defined in (c),

(e) $-CH_2NL_2L_3$, wherein $L_2$ and $L_3$ are hydrogen or $(C_1-C_4)$-alkyl, being the same or different, or the pharmacologically acceptable acid addition salts thereof when $N_1$ is $-CH_2NL_2L_3$,

(f) $-CO_2R_1$,

(g) $-CH_2OH$,

(h) $-CONR_5R_6$,

(i) $-CH_2-NR_5R_6$, or

(j) $-CONHSO_2CH_3$;

wherein $M_1$ is cis or trans $-CH=CH-(CH_2)_m-$, $-(CH_2)_n-$, $-(CH_2)_{n-1}-CF_2-$, $-CO-(CH_2)_3-CH_2-$, trans-$(CH_2)_m-CH=CH-$; $-CH=CH-(CH_2)_{m-1}-CF_2-$, or $-CO-(CH_2)_3-CF_2-$;

wherein $L_1$ is $\alpha$-OH:$\beta$-H, $=CH_2$, or oxo;

wherein $R_8$ is hydrogen, hydroxy, or methyl;

wherein $E_1$ is trans-$CH=CH$, $-CH_2-CH_2-$, or $-C\equiv C-$;

wherein $Q_1$ is $\alpha$-OH:$\beta$-$R_2$, $\alpha$-$R_2$:$\beta$-OH, or oxo;

wherein $A_1$ is $\alpha$-H:$\beta$-$CH_3$, $\alpha$-$CH_3$:$\beta$-H; $\alpha$-H:$\beta$-H, $\alpha$-F:$\beta$-F or $\alpha$-$CH_3$:$\beta$-$CH_3$;

wherein $R_7$ is:

1) $-(CH_2)_p-CH_3$,

2) cis-$CH=CH-CH_2-CH_3$,

3) $-(CH_2)_2-C\equiv CH$,

4) $-(CH_2)_3-CH=C(CH_3)_2$, or

5) $-C(R_3R_4)-(CH_2)_g-CH_2R_{14}$;

wherein $R_2$ is hydrogen or methyl;

wherein $R_3$ and $R_4$ are the same or different and are hydrogen or methyl;

wherein $R_5$ and $R_6$ are the same or different and are hydrogen, methyl, or ethyl;

wherein $R_{14}$ is hydrogen or $(C_1-C_4)$alkyl;

wherein g is an integer of from one to 4, inclusive;

wherein m is an integer of from 3 to 5, inclusive;

wherein n is an integer of from 5 to 7, inclusive; and

wherein p is an integer from one to 6, inclusive or the pharmacologically acceptable salts thereof when $N_1$ is $-CONR_{53}SO_2R_{51}$.

With regard to the divalent substituents described above (e.g., $L_1$, $Q_1$ and $A_1$), these divalent radicals are defined as $\alpha$-$R_i$:$\beta$-$R_j$, wherein $R_i$ represents the substituent of the divalent moiety in the alpha configuration with respect to the plane of the C-8 to C-12 cyclopentane ring and $R_j$ represents the substituent of the divalent moiety in the beta configuration with respect to the plane of the ring. Accordingly, when $L_1$ is defined as $\alpha$-OH:$\beta$-H, the hydroxy of the $L_1$ moiety is in the alpha configuration and the hydrogen substituent is in the beta configuration.

The carbon atom content of various hydrocarbon-containing moieties is indicated by a prefix designating the minimum and maxiumum number of carbon aoms in the moiety, i.e., the prefix $(C_i-C_j)$ indicates a moiety of the integer "i" to the integer "j" carbon atoms, inclusive. Thus $(C_1-C_3)$alkyl refers to alkyl of one to 3 carbon atoms, inclusive, or methyl, ethyl, propyl, and isopropyl.

When $Q_1$ contains a methyl substituent the compounds are all namedas "15-methyl" compounds. Further, when the $E_1$ moiety contains an hydroxyl in the beta configuration, such compounds are additionally named as "15-epi" compounds. The prostaglandin compounds herein which contain $-(CH_2)_2-$, or -C≡C- as the $E_1$ moiety, are accordingly referred to as "13,14-dihydro", or "13,14-didehydro" compounds, respectively.

When $R_7$ is straight chained $-(CH_2)_pCH_3$ wherein p is as defined above, the compounds so described are named as "19,20-dinor", "20-nor", "20-methyl" or "20-ethyl" compounds when p is one, 2, 4, or 5, respectively. When $R_7$ is branched chain $-C(R_3R_4)-(CH_2)_g-CH_2R_{14}$, then the compounds so described are "17-, 18-, 19-, or 20-alkyl" or "17,17-, 17,18-, 17,19-, 17,20-, 18,18-, 18,19-, 18,20-, 19,19-, or 19,20-dialkyl" compounds when g is 4 or 5 and the unbranched portion of the chain is at least n-butyl, e.g., "17,20-dimethyl" compounds are descibed when g is 5 (1-methylpentyl).

When $R_7$ is cis-CH=CH-$CH_2CH_3$, the compounds so described are named as "cis-17,18-didehydro" compounds.

When $R_7$ is $-(CH_2)_3-CH=C(CH_3)_2$, the compounds so described are named as "20-isopropylidene" compounds.

When $N_1$ is $-COL_4$, the compounds herein are named as amides. Further, when $N_1$ is $-COOR_1$, the compounds herein are named as prostaglandin esters and salts.

Examples of amides herein (i.e., $N_1$ is $-COL_4$) include the following:

(1) Amides within the scope of alkylamino groups of the formula-$NR_{51}R_{52}$ are methylamide, ethylamide, n-propylamide, n-butylamide, n-pentylamide, n-hexylamide, n-heptylamide, n-octylamide, n-nonylamide, n-decylamide, n-undecylamide, and n-dodecylamide, and isomeric forms thereof. Further examples are dimethylamide, diethylamide, di-n-propylamide, di-n-butylamide, methylethylamide, methylpropylamide, methylbutylamide, ethylpropylamide, ethylbutylamide, and propylbutylamide. Amides within the scope of cycloalkylamino are cyclopropylamide, cyclobutylamide, cyclopentylamide, 2,3-dimethylcyclopentylamide, 2,2-dimethylcyclopentylamide, 2-methylcyclopentylamide, 3-tert-butylcyclopentyl amide, cyclohexylamide, 4-tert-butylcyclohexylamide, 3-isopropylcyclohexylamide, 2,2-dimethylcyclohexylamide, cycloheptylamide, cyclooctylamide, cyclononylamide, cyclodecylamide, N-methyl-N-cyclobutylamide, N-methyl-N-cyclopentylamide, N-methyl-N-cyclohexylamide, N-ethyl-N-cyclopentylamide, and N-ethyl-N-cyclohexylamide. Amides within the scope of aralkylamino are benzylamide, 2-phenylethylamide, and N-methyl-N-benzyl-amide. Amides within the scope of substituted phenylamide are p-chloroanilide, m-chloroanilide, 2,4-dichloroanilide, 2,4,6-trichloroanilide, m-nitroanilide, p-nitroanilide, p-methoxyanilide, 3,4-dimethoxyanilide, 3,4,5-trimethoxyanilide, p-hydroxymethylanilide, p-methylanilide, m-methylanilide, p-ethylanilide, t-butylanilide, p-carboxyanilide, p-methoxycarbonyl anilide, p-carboxyanilide and o-hydroxyanilide. Amides within the scope of carboxyalkylamino are carboxyethylamide, carboxypropylamide and carboxymethylamide, carboxybutylamide. Amides within the scope of carbamoylalkylamino are carbamoylmethylamide, carbamoylethylamide, carbamoylpropylamide, and carbamoylbutylamide. Amides within the scope of cyanoalkylamino are cyanomethylamide, cyanoethylamide, cyano

propylamide, and cyanobutylamide. Amides within the scope of acetylalkylamino are acetylmethylamide, acetylethylamide, acetylpropylamide, and acetylbutylamide. Amides within the scope of benzoylalkylamino are benzoylmethylamide, benzoylethylamide, benzoylpropylamide, and benzoylbutylamide. Amides within the scope of substituted benzoylalkylamino are p-chlorobenzoylmethylamide, m-chlorobenzoylmethylamide, 2,4-dichlorobenzoylmethylamide, 2,4,6-trichlorobenzoylmethylamide, m-nitrobenzoylmethylamide, p-nitrobenzoylmethylamide, p-methoxybenzoylmethylamide, 2,4-dimethoxybenzoylmethylamide, 3,4,5-trimethoxybenzoylmethylamide, p-hydroxymethylbenzoylmethylamide, p-methylbenzoylmethylamide, m-methylbenzoylmethylamide, p-ethylbenzoylmethylamide, t-butylbenzoylmethylamide, p-carboxybenzoylmethylamide, m-methoxycarbonylbenzoylmethylamide, o-carboxybenzoylmethylamide, o-hydroxybenzoylmethylamide, p-chlorobenzoylethylamide, m-chlorobenzoylethylamide, 2,4-dichlorobenzoylethylamide, 2,4,6-trichlorobenzoylethylamide, m-nitrobenzoylethylamide, p-nitrobenzoylethylamide, p-methoxybenzoylethylamide, p-methoxybenzoylethylamide, 2,4-dimethoxybenzoylethylamide, 3,4,5-trimethoxybenzoylethylamide, p-hydroxymethylbenzoylethylamide, p-methylbenzoylethylamide, m-methylbenzoylethylamide, p-ethylbenzoylethylamide, t-butylbenzoylethylamide, p-carboxybenzoylethylamide, m-methoxycarbonylbenzoylethylamide, o-carboxybenzoylethylamide, o-hydroxybenzoylethylamide, p-chlorobenzoylpropylamide, m-chlorobenzoylpropylamide, 2,4-dichlorobenzoylpropylamide, 2,4,6-trichlorobenzoylpropylamide, m-nitrobenzoylpropylamide, p-nitrobenzoylpropylamide, p-methoxybenzoylpropylamide, 2,4-dimethoxybenzoylpropylamide, 3,4,5-trimethoxybenzoylpropylamide, p-hydroxymethylbenzoylpropylamide, p-methylbenzoylpropylamide, m-methylbenzoylpropylamide, p-ethylbenzoylpropylamide, t-butylbenzoylpropylamide, p-carboxybenzoylpropylamide, m-methoxycarbonylbenzoylpropylamide, o-carboxybenzoylpropylamide, o-hydroxybenzoylpropylamide, p-chlorobenzoylbutylamide, m-chlorobenzoylbutylamide, 2,4-dichlorobenzoylbutylamide, 2,4,6-trichlorobenzoylbutylamide, m-nitrobenzoylmethylamide, p-nitrobenzoylbutylamide, p-methoxybenzoylbutylamide, 2,4-dimethoxybenzoylbutyl-amide, 3,4,5-trimethoxybenzoylbutylamide, p-hydroxymethylbenzoylbutyl-amide, p-methylbenzoylbutyamide, m-methylbenzoylbutylamide, p-ethyl-benzoylbutyalmide, m-methylbenzoylbutylamide, p-ethylbenzoylbutyl-amide, t-butylbenzoylbutylamide, p-carboxybenzoylbutylamide, m-methoxycarbonylbenzoylbutylamide, o-carboxy-

benzoylbutylamide, o-hydroxybenzoylmethylamide. Amides within the scope of pyridylamino are α-pyridylamide, β-pyridylamide, and γ-pyridylamide. Amides within the scope of substituted pyridylamino are 4-methyl-α-pyridylamide, 4-methyl-β-pyridylamide, 4-chloro-α-pyridylamide, and 4-chloro-β-pyridylamide. Amides within the scope of pyridylalkylamino are α-pyridylmethylamide, β-pyridylmethylamide, γ-pyridylmethylamide, α-pyridylethylamide, β-pyridylethylamide, γ-pyridylethylamide, α-pyridylpropylamide, β-pyridylpropylamide, γ-pyridylpropylamide, α-pyridylbutylamide, β-pyridylbutylamide, and γ-pyridylbutylamide. Amides within the scope of substituted pyridylalkylamido are 4-methyl-α-pyridylmethylamide, 4-methyl-β-pyridylmethylamide, 4-chloro-α-pyridylmethylamide, 4-chloro-β-pyridylmethyl-amide, 4-methyl-α-pyridylpropylamide, 4-methyl-β-pyridylpropylamide, 4-chloro-α-pyridylpropylamide, 4-chloro-β-pyridylpropylamide, 4-methyl-α-pyridylbutylamide, 4-methyl-β-pyridylbutylamide, 4-chloro-α-pyridylbutylamide, 4-chloro-β-pyridylbutylamide. Amides within the scope of hydroxyalkylamino are hydroxymethylamide, β-hydroxyethylamide, β-hydroxypropylamide, γ-hydroxypropylamide, 1-(hydroxymethyl)ethylamide, 1-(hydroxymethyl)propylamide, (2-hydroxymethyl)propylamide, and α,α,-dimethyl-hydroxyethylamide. Amides within the scope of dihydroxyalkylamino are dihydroxymethylamide, β,γ-dihydroxypropylamide, 1-(hydroxymethyl)2-hydroxymethylamide, β,γ-dihydroxybutylamide, β,δ-dihydroxybutyl-amide, γ,δ-dihydroxybutylamide, and 1,1-bis(hydroxymethyl)-ethylamide. Amides within the scope of trihydroxyalkylamino are tris-(hydroxy-methyl)-methylamide and 1,3-dihydroxy-2-hydroxymethylpropylamide.

(2) Amides within the scope of cycloamino groups described above are pyrrolidylamide, piperidylamide, morpholinylamide, hexamethylene iminylamide, piperazinylamide, pyrrolinylamide, and 3,4-didehydropiperidinylamide.

(3) Amides within the scope of carbonylamino of the formula -NR53COR51 are methylcarbonylamide, ethylcarbonylamide, phenylcarbonylamide, and benzylcarbonylamide.

(4) Amides within the scope of sulfonylamino of the formula -NR53SO2R51 are methylsulfonylamide, ethylsufonylamide, phenylsulfonylamide, p-tolylsulfonylamide, benzylsulfonylamide.

Examples of alkyl of one to 12 carbon atoms, inclusive, are methyl,

ethyl, propyl, butyl, pentyl, hexyl, heptyl, octyl, nonyl, decyl, undecyl, dodecyl, isomeric forms thereof.

Examples of ($C_3$-$C_{10}$)cycloalkyl which includes alkyl-substituted cycloalkyl, are cyclopropyl, 2-methylcyclopropyl, 2,2-dimethylcyclopropyl, 2,3-diethylcyclopropyl, 2-butylcyclopropyl, cyclobutyl, 2-methylcyclobutyl, 3-propylcyclobutyl, 2,3,4-triethylcyclobutyl, cyclopentyl, 2,2-dimethylcyclopentyl, 2-pentylcyclopentyl, 3-tert-butylcyclopentyl, cyclohexyl, 4-tert-butylcyclohexyl, 3-isopropylcyclohexyl, 2,2-dimethylcyclohexyl, cycloheptyl, cyclooctyl, cyclononyl, and cyclodecyl.

Examples of ($C_7$-$C_{12}$)aralkyl are benzyl, 2-phenylethyl, 1-phenylethyl, 2-phenylpropyl, 4-phenylbutyl, 3-phenylbutyl, 2-(1-naphthylethyl), and 1-(2-naphthylmethyl).

Examples of phenyl substituted by one to 3 chloro or alkyl of one to 4 carbon atoms, inclusive, are p-chlorophenyl, m-chlorophenyl, 2,4-dichlorophenyl, 2,4,6-trichlorophenyl, p-tolyl, m-tolyl, o-tolyl, p-ethylphenyl, p-tert-butylphenyl, 2,5-dimethylphenyl, 4-chloro-2-methylphenyl, and 2,4-dichloro-3-methylphenyl.

Examples of ($C_5$-$C_7$)cycloalkyl optionally substituted by ($C_1$-$C_4$)alkyl are cyclobutyl, 1-propylcyclobutyl, 1-butylcyclobutyl, 1-pentylcyclobutyl, 2-methylcyclobutyl, 2-propylcyclobutyl, 3-ethylcyclobutyl, 3-propylcyclobutyl, 2,3,4-triethylcyclobutyl, cyclopentyl, 2,2-dimethylcyclopentyl, 3-ethylcyclopentyl, 3-propylcyclopentyl, 3-butylcyclopentyl, 3-tert-butylcyclopentyl, 1-methyl-3-propylcyclopentyl, 2-methyl-3-propylcyclopentyl, 2-methyl-4-propylcyclopentyl, cyclohexyl, 3-ethylcyclohexyl, 3-isopropylcyclohexyl, 4-methylcyclohexyl, 4-ethylcyclohexyl, 4-propylcyclohexyl, 4-butylcyclohexyl, 4-tert-butylcyclohexyl, 2,6-dimethylcyclohexyl, 2,2-dimethylcyclohexyl, 2,6-dimethyl-4-propylcyclohexyl, and cycloheptyl.

Pharmacologically acceptable cations within the scope of $R_1$ include the pharmacologically acceptable metal cations, ammonium, amine cations, or quaternary ammonium cations.

Especially preferred metal cations are those derived from the alkali metals, e.g., lithium, sodium, and potassium, and from the alkaline earth metals, e.g., magnesium and calcium, although cationic forms of other metals, e.g., aluminum, zinc, and iron are within the scope of this invention.

Pharmacologically acceptable amine cations are those derived from primary, secondary, or tertiary amines. Examples of suitable amines are methylamine, dimethylamine, trimethylamine, ethylamine, dibutylamine, triisopropylamine, N-methylhexylamine, decylamine, dodecylamine, allylamine, crotylamine, cyclopentylamine, dicyclohexylamine, benzylamine, dibenzylamine, α-phenylethylamine, β-phenylethylamine, ethylenediamine, diethylenetriamine, and the like aliphatic, cycloaliphatic, araliphatic amines containing up to and including about 18 carbon atoms, as well as heterocyclic amines, e.g., piperidine, morpholine, pyrrolidine, piperazine, and lower-alkyl derivatives thereof, e.g., 1-methylpiperidine, 4-ethylmorpholine, 1-isopropylpyrrolidine, 2-methylpyrrolidine, 1,4-dimethylpiperazine, 2-methylpiperidine, and the like, as well as amines containing water-solubilizing or hydrophilic groups, e.g, mono-, di-, and triethanolamine, ethyldiethanolamine, N-butylethanolamine, 2-amino-1-butanol, 2-amino-2-ethyl-1,3-propanediol, 2-amino-2-methyl-1-propanol, tris(hydroxymethyl)aminomethane, N-phenylethanolamine, N-(p-tert-amylphenyl)-diethanolamine, glactamine,- N-methylglycamine, N-methylglucosamine, ephedrine, phenylephrine, epinephrine, procaine, and the like. Further useful amine salts are the basic amino acid salts, e.g., lysine and arginine.

Examples of suitable pharmacologically acceptable quaternary ammonium cations are tetramethylammonium, tetraethylammonium, benzyltrimethylammonium, phenyltriethylammonium, and the like.

In accordance with the manner and process of using the present invention, a $PGE_1$-like prostaglandin in pharmaceutical dosage form is administered systemically to humans to treat or prevent ARDS or multiple organ failure as described herein. The terms "shock", "trauma", and "sepsis" are used in their ordinary accepted meaning in the art. Shock may be generally described as a condition of acute circulatory failure due to derangement of circulatory control or loss of circulating fluid. It is frequently marked by palor and clamminess of the skin, decreased blood pressure, feeble rapid pulse, decreased respiration, restlessness, anxiety, and sometimes unconsciousness. Trauma may be generally described as any wound or injury. This can be the result of surgery. Sepsis may be generally described as a serious bacterial infection with a concurrent, deleterious systemic response. The term

"ARDS" is well defined in the medical literature, as described above. It is caused by well known factors, for example those noted above and in Table I. All of these terms are well-known to physicians of ordinary skill.

By "severe vital organ failure" is meant a decrease in functioning of a vital organ (gastrointestinal tract, brain, liver, kidney, lung, or heart) to the extent that the chance of survivability is low. By "humans having or prone to vital organ failure" is meant a human experiencing or recently experiencing a catastrophic event such as trauma, sepsis, shock or other events placing one at risk for ARDS or vital organ failure such as those listed above and in Table 1. These persons are readily identified by a skilled physician, using, e.g., these factors and the clinical symptoms set forth above.

By "unit dose" is meant a discrete quantity of a prostaglandin and a carrier useful for systemic administration in the method of this invention. Thus, an ideal unit dose would be one wherein one unit, or an integral amount thereof, contains the precise amount of prostaglandin for this purpose. These unit doses can be packaged in a variety of forms. The prostaglandin may be dissolved or suspended in the carrier, or the prostaglandin and the carrier may be combined just prior to administration.

Accordingly, the prostaglandin in pharmaceutical dosage form is systemically administered, preferably intravenously, to the patient at a rate of about 1 to 100 ng/kg/min for $PGE_1$-like compounds of the Formula I and 0.1 to 10 ng/kg/min for $PGI_2$ its salts, esters, amides, and stabilized analogs, respectively, for a period of about 2-14 days or until the patient is extubated, or until the patient is no longer threatened by the organ failure. The preferred rate is 20 to 30 ng/kg/min and 2 to 5 ng/kg/min, respectively, over a period of about 7 days.

$PGE_1$, its alkyl esters and salts are well known, readily available compound and can be prepared by methods known in the art. U.S. Patent 3,069,322 discloses such methods. The essential material constituting a disclosure of how to prepare $PGE_1$, its lower alkyl esters and salts are incorporated by reference from U.S. Patent 3,069,322. Amides of $PGE_1$ can be prepared by methods known in the art for converting carboxylic acid moieties to carboxylic acid amide moieties. Such

methods are described, for example, in U.S. Patents 3,954,741 and 4,100,192 (columns 10 and 11). Both of these patents are expressly incorporated by reference herein.

The other prostaglandins within the scope of this invention are known and can be synthesized by the methods described in the U.S. patents noted in the Information Disclosure statement above, which are expressly incorporated by reference herein.

$PGI_2$ and its pharmaceutical salts, esters and amides can be prepared by the methods disclosed in U.S. Patents 4,158,667 and 4,338,325. Stabilized analogs thereof are described in U.S. Patents 4,238,414 and 4,420,632. The essential material constituting a disclosure of how to prepare prostacyclin, its pharmaceutical salts, and stabilized analogs are incorporated by reference from these U.S. Patents.

In accomplishing the purposes of this invention those compounds which are useful are those prostaglandins and prostaglandin derivatives which are at least 1% as potent as $PGE_1$ in preventing and/or ameliorating the deleterious consequences of trauma, sepsis, shock or other traumatic events listed in Table 1 under the conditions of the Examples. These compounds are referred to herein as $PGE_1$-like compounds because of their similar biological activity. Examples of such compounds include $PGI_2$, and salts, esters, amides and stabilized analogs thereof (including carbacyclin and 9β-methyl-carbacyclin), and those prostaglandins of the Formula I described above, particularly:

$PGE_1$;
(15S)-15-methyl-$PGE_1$;
(15R)-15-methyl-$PGE_1$;
16,16-dimethyl-$PGE_1$;
2-decarboxy-2-hydroxymethyl-$PGE_1$;
$PGE_2$;
15-keto-$PGE_2$;
16,16-dimethyl-$PGE_2$;
17S,20-dimethyl-6-oxo-$PGE_1$, methyl ester;
17S,20-dimethyl-trans-delta-2-$PGE_1$;
$PGE_2$, N-methanesulfonylamide;
9-deoxo-9-methylene-16,16-dimethyl-$PGE_2$;
(15S)-15-methyl-$PGE_2$;

(15R)-15-methyl-$PGE_2$;

11-deoxy-16,16-dimethyl-$PGE_2$;

11-deoxy-11α-16,16-trimethyl-$PGE_2$;

6-oxo-11-deoxy-11α,16,16-trimethyl-$PGE_2$;

6-oxo-$PGE_2$;

6-oxo-$PGE_1$;

11-deoxy-15-methyl-$PGE_1$;

$PGE_3$;

16,16-difluoro-$PGE_2$;

and

20-isopropylidene-$PGE_1$

as well as the alkyl esters, pharmacologically acceptable salts and derivatives thereof. These compounds may also be in stabilized forms including clathrates (see, e.g., U.S. Patent 4,254,736), triacetin (see U.S. Patent 3,966,962) or triacetin/ethanol (see U.S. Patent 4,301,175) formulations, triacetin/silicon dioxide gels (see copending application S.N. 613,006, filed 21 May 1984), and the like. These patents are incorporated by reference herein.

$PGE_1$ is the most preferred compound of this invention.

Pharmaceutical dosage forms of $PGE_1$-like compounds here for intravenous infusions can be formulated by known conventional methods. Other dosage forms of these compounds are also well known.

$PGE_1$, also known as alprostadil, is the active ingredient of Prostin VR Pediatric, which is sold by The Upjohn Company to aid in the maintenance of patency of the ductus arteriosis in neonates. See, Physicians Desk Reference, p. 2043 (38th Ed. 1984). This sterile solution of $PGE_1$ is most preferred for use in the method of the instant invention. A similar formulation can be used for other prostaglandins.

For the treatment or prevention of ARDS or organ failure the $PGE_1$-like prostaglandin in a pharmaceutical dosage form is preferably administered through a central venous catheter or through the right atrial port of a Swan-Ganz catheter, and the dosage is increased until a dose of 30 ng/kg/min is reached. The dosage is calculated on the basis of the patient's pre-illness weight. A preferred method of administration is to begin with an infusion rate of 5 ng/kg/min and to increase the dose by 5 ng/kg/min every 30 minutes until a rate

of 30 ng/kg/min is obtained. The prostaglandin is given for a total of seven days, until the patient is extubated, or as long as the patient is threatened by organ failure. The infusion rate of 1 to 100 ng/kg/min is maintained as long as the mean systemic arterial pressure does not decrease more than twenty (20%) percent from baseline levels. If there is a decrease of more than 20% in the arterial pressure, the infusion rate is decreased until levels within 20% of baseline levels are reached again. Discontinuing the drug preferably proceeds by decreasing the dosage 10 ng/kg/min per hour.

Equivalent dosages for other $PGE_1$-like prostaglandins are also employed, based on the compound's potency as compared to $PGE_1$.

The identical procedure is utilized for the treatment or prevention of ARDS and/or multiple organ failure with $PGI_2$ and salts, esters, amides, and stabilized analogs thereof, except that the dosage is 0.1 to 10 ng/kg/min., preferably 2 to 5 ng/kg/min.

The intravenous route is preferred for $PGE_1$-like prostaglandins (including $PGI_2$, its salts, esters, and amides). However, other systemic and non-systemic routes of administration (e.g., oral, enteral, rectal, parenteral including via implanted devices, intra-arterial, cutaneous as via patches) may also be employed as long as the dosage used achieves the same blood level of drug or equivalent effect as the intravenous route. A convenient route may be into the right atrium through the right atrial port of a Swan-Ganz catheter, if such a catheter is in place.

The following Examples are illustrative of the uses (uses) of this invention, but are not to be construed as limiting.

Example 1 Treating Severe Lung Failure with $PGE_1$

An intravenous solution was prepared by mixing one 5 ml ampoule of Prostin VR Pediatric Sterile Solution containing 2.5 mg $PGE_1$ active drug with one liter of $D_5W$ or Normal Saline. Dosages of this solution or of a control solution containing only vehicle but no $PGE_1$ were administered to 41 patients suffering from ARDS in a prospective randomized double blind placebo controlled study. $PGE_1$ or the control solution was started at a dose of 5 ng/kg/min and then increased 5 ng/kg/min every 30 minutes until a rate of 30 ng/kg/min was reached.

This dosage was continued for 7 days or until the patient was extubated, whichever came sooner. The survival rate 30 days after discontinuing the infusion was as follows:

| Treatment | Number of Patients | Survival Rate (%) |
|---|---|---|
| $PGE_1$ | 21 | 71.4 |
| Control | 20 | 35.0 |

Example 2 Treating Severe Organ Failure with $PGE_1$

Fifty patients with ARDS were randomized to receive $PGE_1$ (30 ng/kg/min for 7 days) or placebo (equivalent volumes of saline and time) in a double blind study. For entry, patients must have required mechanical ventilation with an inspired oxygen concentration of 40% or more and a positive end respiratory pressure of 5 cm of water or more. Most of the patients (70%) were septic. 17 out of 26 patients who had received $PGE_1$ survived (65% survival) while only 7 out of 24 patients who had received placebo survived (29% survival).

The relation between the bilirubin and creatinine levels and the mortality of the patients enrolled in this study is depicted in Table 2. As can be seen, there is a significantly higher survival rate for those patients who were treated with $PGE_1$ while their creatinine and bilirubin levels are in the normal range. Creatinine and bilirubin levels are indicative of kidney and liver function, respectively. The data thus indicate that $PGE_1$ prevents severe kidney and liver damage, and greatly decreases the risk of death, for patients highly susceptible to kidney failure, liver failure, and/or death as a result of trauma, sepsis, shock, and similar catastrophic events.

Example 3 Preventing Organ Failure with $PGE_1$

A patient with peritonitis diagnosed at surgery has a significant risk of developing multiple organ failure (20-30%). The patient has an intravenous line placed so that its tip resides in the subclavian vein. A solution of $PGE_1$ in normal saline (2.5 mg in 1 l saline) is infused at the rate of 5 ng/kg/min. The dose is increased at the rate of 5 ng/kg/min every 30 minutes until a maximum infusion rate of 30 ng/kg/min is achieved. This dosage is continued for 7 days to prevent the development of severe organ failure, including ARDS. During this period the peritonitis is brought under control surgically as needed and with antibiotic therapy.

Example 4 Preventing Organ Failure with $PGE_2$

A patient is involved in a motor car accident and receives severe damage to the abdominal region with massive internal bleeding. During the period prior to shortly after surgical repair of the abdominal injuries a total of 8 units of blood were required to maintain an adequate blood volume. This subjects the patient to significant risk for the development of organ failure. An intravenous line is established in the subclavian vein and $PGE_2$ in normal saline (2.5 mg in 1 l saline) is infused at the rate of 5 ng/kg/min. This dose is increased at the rate of 5 ng/kg/min every 30 minutes to a maximum of 30 ng/kg/min. This dosage is continued for 5 days to prevent the development of vital organ dysfunction.

Example 5 Preventing Organ Failure with $PGI_2$

A patient is admitted to the intensive care unit with labored breathing following surgery. During induction of anesthesea, the patient aspirates stomach contents into the lungs which results in pulmonary insufficiency. A venous line is established and a solution of prostacyclin ($PGI_2$) is infused at the rate of 2.5 ng/kg/min. This infusion rate is maintained for 7 days to prevent the development of vital organ (liver, kidney, lung, heart) failure. The patient is retained for an additional 2 days in the intensive care unit (ICU).

Example 6 Preventing Organ Failure with $PGE_1$

A patient is admitted to the hospital emergency room following a fall of 8 stories from a building under construction. He has six fractured ribs, a fractured tibia and multiple contusions. The broken tibia is set and the patient is held for observation. During the fifth day his blood bilirubin levels, which had been rising, are at 6 mg/dl and his creatinine levels are 2.2 mg/dl. Infusion of a solution of $PGE_1$ in dextrose is started into the subclavian vein at the rate of 5 ng/kg/min and increased over a period of 2 hours to 30 ng/kg/min. This dosage is continued for 7 days to prevent further organ damage from occurring.

TABLE 1

CONDITIONS ASSOCIATED WITH ARDS

Shock
- Hemorrhagic
- Hypovolemic
- Traumatic
- Septic
- Cardiogenic
- Cadiac Compressive
- Anaphylactic
- Neurogenic
- Toxic

Trauma
- Fat Emboli
- Lung Contusion
- Nonthoracic Trauma

Infection
- Viral Pneumonia
- Bacterial Pneumonia
- Fungal Pneumonia

Inhalation of Toxic Gases
- Oxygen ($FIO_2$>0.5)
- Smoke
- Combustion Products
- Nitrous Oxide
- Industrial Solvents

Aspiration of Gastric Contents
- Especially with a pH <2.5

Drug Overdose
- Heroin
- Methadone
- Barbiturates
- Ethchlorvynol

Disseminated Intravascular Coagulation

Near Drowning
- Fresh or Salt Water

Miscellaneous
- Pancreatitis
- Uremia
- Postcardiopulmonary Bypass
- Postcardioversion
- Air or Amniotic Fluid Emboli
- Multiple Transfusions

TABLE 2

| | Percent Mortality (N) | |
|---|---|---|
| | $PGE_1$ | Placebo |
| Normal creatinine | 18 (17) | 65 (17) |
| Normal bilirubin | 15 (13) | 44 (9) |
| Normal creatinine and bilirubin | 9 (11) | 44 (9) |
| Abnormal creatinine* | 67 (9) | 83 (6) |
| Abnormal bilirubin | 58 (12) | 86 (14) |
| Abnormal creatinine and bilirubin* | 71 (7) | 83 (6) |

*Abnormal = >1.5 mg/dl

N = number of patients in study

CLAIMS

1. The use of a PGE-like prostaglandin for the manufacture of a medicament for the treatment of a human suffering from acute respiratory distress syndrome or multiple vital organ damage.

2. The use of a PGE-like prostaglandin for the manufacture of a medicament for the prevention of acute respiratory distress syndrome or multiple vital organ damage in a human prone to the syndrome or organ damage.

3. The use of a PGE-like prostaglandin for the manufacture of a medicament for the treatment of a human suffering from shock, trauma, sepsis or any combination thereof.

4. The use of a PGE-like prostaglandin for the manufacture of a medicament for reducing the probability of a fatal outcome for a human suffering from or prone to shock, trauma, sepsis or any combination thereof.

5. A use according to claim 1, for the prevention of multiple vital organ damage.

6. A use according to any preceding claim, wherein the medicament is suitable for administration for at least 7 days.

7. A use according to any preceding claim, wherein the prostaglandin has the formula

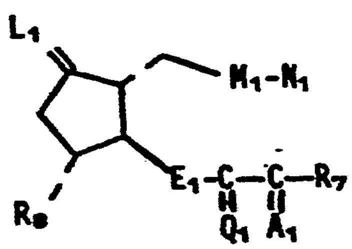

I

wherein $N_1$ is

(1) $-COOR_1$, wherein $R_1$ is

(a) hydrogen,

(b) $(C_1-C_{12})$alkyl,

(c) $(C_1-C_{10})$cycloalkyl,

(d) $(C_6-C_{16})$aralkyl,

(e) phenyl, optionally substituted by one, 2 or 3 substituents independently selected from chloro and $(C_1-C_3)$alkyl,

(f) phenyl substituted in the para position by (i) $-NH-CO-R_{25}$, (ii) $-CO-R_{26}$, (iii) $-O-CO-R_{54}$ or (iv) $-CH=N-NH-CO-NH_2$, wherein

$R_{25}$ is methyl, phenyl, acetamidophenyl, benzamidophenyl or $-NH_2$; $R_{26}$ is methyl, phenyl, $-NH_2$ or methoxy; and $R_{54}$ is phenyl or acetamidophenyl; or

(g) a pharmacologically acceptable cation;

(2) $-CH_2OH$,

(3) $-COL_4$, wherein $L_4$ is

(a) amino of the formula $-NR_{51}R_{52}$ wherein $R_{52}$ is

(i) hydrogen or

(ii) $(C_1-C_{12})$alkyl,

and $R_{51}$ is

(i) hydrogen,

(ii) $(C_1-C_{12})$alkyl,

(iii) $(C_3-C_{10})$cycloalkyl,

(iv) $(C_7-C_{12})$aralkyl,

(v) phenyl optionally substituted by one, 2 or 3 substituents independently selected from chloro, $(C_1-C_3)$alkyl, hydroxy, carboxy, $(C_2-C_5)$alkoxycarbonyl and nitro,

(vi) $(C_2-C_5)$carboxyalkyl,

(vii) $(C_2-C_5)$carbamoylalkyl,

(viii) $(C_2-C_5)$cyanoalkyl,

(ix) $(C_3-C_6)$acetylalkyl,

(x) $(C_7-C_{11})$benzoalkyl optionally substituted by one, 2 or 3 substituents independently selected from chloro, $(C_1-C_3)$alkyl, hydroxy, $(C_1-C_3)$alkoxy, carboxy, $(C_2-C_5)$alkoxycarbonyl and nitro,

(xi) pyridyl optionally substituted by one, 2 or 3 substituents independently selected from chloro, $(C_1-C_3)$alkyl and $(C_1-C_3)$alkoxy,

(xii) $(C_6-C_9)$pyridylalkyl optionally substituted by one, 2 or 3 substituents independently selected from chloro and $(C_1-C_3)$alkyl,

(xiii) $(C_1-C_4)$hydroxyalkyl,
(xiv) $(C_2-C_4)$dihydroxyalkyl or
(xv) $(C_3-C_4)$trihydroxyalkyl,

(b) cycloamino selected from pyrrolidino, piperidino, morpholino, piperazino, hexamethyleneimino, pyrrolino and 3,4-didehydropiperidinyl optionally substituted by one or 2 $(C_1-C_{12})$alkyl groups,

(c) carbonylamino of the formula $-NR_{53}COR'_{51}$, wherein $R_{53}$ is H or $(C_1-C_4)$alkyl and $R'_{51}$ is as defined for $R_{51}$ but is not hydrogen,

(d) sulfonylamino of the formula $-NR_{53}SO_2R'_{51}$, wherein $R'_{51}$ and $R_{53}$ are as defined above,

(e) $-CH_2NL_2L_3$, wherein $L_2$ and $L_3$ are independently selected from H and $(C_1-C_4)$alkyl,

(f) $-CO_2R_1$,

(g) $-CH_2OH$,

(h) $-CONR_5R_6$, wherein $R_5$ and $R_6$ are independently selected from H, $CH_3$ and $C_2H_5$,

(i) $-CH_2-NR_5R_6$, or

(j) $-CONHSO_2CH_3$;

wherein $M_1$ is cis or trans-$CH=CH-(CH_2)_m-$, $-(CH_2)_n-$, $-(CH_2)_{n-1}-CF_2-$, $-CO-(CH_2)_3-CH_2-$, trans-$(CH_2)_m-CH=CH-$, $-CH=CH-(CH_2)_{m-1}-CF_2-$ or $-CO-(CH_2)_3-CF_2-$, m being 3, 4 or 5 and n being 5, 6 or 7;

wherein $L_1$ is $\alpha$-OH:$\beta$-H, $=CH_2$ or oxo;

wherein $R_8$ is hydrogen, hydroxy or methyl;

wherein $E_1$ is trans-CH=CH, $-CH_2CH_2-$ or $-C\equiv C-$;

wherein $Q_1$ is $\alpha$-OH:$\beta$-H, $\alpha$-OH:$\beta$-$CH_3$, $\alpha$-H:$\beta$-OH, $\alpha$-$CH_3$:$\beta$-OH or oxo;

wherein $A_1$ is $\alpha$-H:$\beta$-$CH_3$, $\alpha$-$CH_3$:$\beta$-H, $\alpha$-H:$\beta$-H, $\alpha$-F:$\beta$-F or $\alpha$-$CH_3$:$\beta$-$CH_3$; and

wherein $R_7$ is

1) $-(CH_2)_p-CH_3$, p being from 1 to 6,

2) cis-$CH=CH-CH_2-CH_3$,

3) $-(CH_2)_2-C\equiv CH$,

4) $-(CH_2)_3-CH=C(CH_3)_2$ or

5) $-C(R_3R_4)-(CH_2)_g-CH_2R_{14}$, g being from 1 to 4, $R_3$ and $R_4$ being independently selected from H and $CH_3$, and $R_{14}$ being H or $(C_1-C_4)$alkyl;

or a pharmacologically acceptable salt thereof.

8. A use according to any of claims 1 to 6, wherein the prostaglandin is selected from:

$PGE_1$;
2-decarboxy-2-hydroxymethyl-$PGE_1$;
(15S)-15-methyl-$PGE_1$;
(15R)-15-methyl-$PGE_1$;
16,16-dimethyl-$PGE_1$;
$PGE_2$;
15-keto-$PGE_2$;
16,16-dimethyl-$PGE_2$;
17S,20-dimethyl-6-oxo-$PGE_1$, methyl ester;
17S,20-dimethyl-trans-delta-2-$PGE_1$;
$PGE_2$, N-methanesulfonylamide;
9-deoxo-9-methylene-16,16-dimethyl-$PGE_2$;
(15S)-15-methyl-$PGE_2$;
(15R)-15-methyl-$PGE_2$;
11-deoxy-16,16-dimethyl-$PGE_2$;
11-deoxy-11α,16,16-trimethyl-$PGE_2$;
6-oxo-11-deoxy-11α,16,16-trimethyl-$PGE_2$;
6-oxo-$PGE_2$;
6-oxo-$PGE_1$;
11-deoxy-15-methyl-$PGE_1$;
$PGE_3$;
16,16-difluoro-$PGE_2$; and
20-isopropylidene-$PGE_1$.

9. A use according to claim 8, wherein the prostaglandin is $PGE_1$.

10. A use according to any of claims 1 to 6, wherein the prostaglandin is $PGE_2$ or $PGI_2$, or a salt, ester, amide or stabilised form or derivative thereof.